**Europäisches Patentamt**

**European Patent Office**

**Office européen des brevets**

(19)

(11) Veröffentlichungsnummer: **0 026 239**
**A1**

(12)

# EUROPÄISCHE PATENTANMELDUNG

(21) Anmeldenummer: **79103713.8**

(22) Anmeldetag: **29.09.79**

(51) Int. Cl.³: **A 61 N 5/06**

(43) Veröffentlichungstag der Anmeldung:
**08.04.81 Patentblatt 81/14**

(84) Benannte Vertragsstaaten:
**AT BE CH DE FR GB IT LU NL SE**

(71) Anmelder: **Köppen, Detlef**
**Heiderhöfen 65**
**D-4200 Oberhausen(DE)**

(72) Erfinder: **Köppen, Detlef**
**Heiderhöfen 65**
**D-4200 Oberhausen(DE)**

(74) Vertreter: **Radünz, Ingo**
**Schumannstrasse 100**
**D-4000 Düsseldorf 1(DE)**

(54) **Medizinisches und kosmetisches Bestrahlungsgerät.**

(57) Es wird ein Bestrahlungsgerät für medizinische und kosmetische Zwecke angegeben, das eine Strahlungsquelle (16) und Vorschaltgeräte zum Betrieb der Strahlungsquelle und eine Filteranordnung (18) enthält, die die kürzerwellige Strahlung unterdrückt, wobei die Kühlung der Strahlungsquelle (16) und des Filters (18) unter Überdruck erfolgt, der von einem Lüfter (12) geliefert wird, und die Strahlungsquelle (16) im Einschaltmoment weniger als bei Vollast gekühlt wird und ein Sicherheitsabschaltsystem vorgesehen ist. Dabei besteht die Strahlungsquelle (16) aus einem Metallhalogenbrenner, der mit verschiedenen Metallen dotiert ist. Aufgrund des vorgesehenen Sicherheitsabschaltsystems wird das Bestrahlungsgerät abgeschaltet, wenn das Filter zerstört und/oder der Lüfter ausfällt.

EP 0 026 239 A1

./...

PATENTANMELDUNG

===========================================================

Anmelder:  Detlef Köppen
           Heiderhöfen 65
           42oo Oberhausen

===========================================================

Titel:     Medizinisches und kosmetisches Bestrahlungsgerät

===========================================================

Die Erfindung betrifft ein medizinisches und kosmetisches
Bestrahlungsgerät.

Mit Hilfe des Bestrahlungsgerätes kann eine Sofortbräunung
und Direktpigmentierung bei einer zu behandelnden Person
erreicht werden. Darüber hinaus eignet sich das Bestrahlungsgerät insbesondere für die Behandlung von Hautkrankheiten,
Akne, usw.

Es ist bereits eine Bestrahlungseinrichtung für fotobiologische und/oder fotochemische Zwecke bekannt (DE-OS
26 09 273), die mindestens eine Ultraviolett-Strahlenquelle und deren zum Betrieb notwendige Geräte zum Erzeugen
der Zündspannung und des Betriebsstromes sowie vorzugsweise
eine Filtervorrichtung enthält, wobei die kürzerwelligen
Strahlen ab 320 Nanometer unterdrückt werden.

Weiter ist eine Bestrahlungseinrichtung für kosmetische,
fotobiologische und/oder fotochemische Zwecke bekannt,
wobei die Kühlung von Ultraviolettstrahlungsquellen und/oder Gehäuse regelbar oder verstellbar ist.

Die bekannten Bestrahlungseinrichtungen weisen im wesentlichen noch Mängel hinsichtlich der Kühlung der Strahlungsquellen und der Absorption der Filtersysteme auf, wobei oftmals zu hohe UV-Bestrahlungsstärken hervorgerufen werden, die sich schädlich auf die Haut der zu behandelnden Person auswirken.

Der Erfindung liegt die Aufgabe zugrunde, ein Bestrahlungsgerät zu schaffen, das eine gute Kühlung der Strahlungsquelle in Verbindung mit einem Filter sicherstellt, das im wesentlichen für die Haut schädliche Strahlung unterdrückt und ein Sicherheitsabschaltsystem aufweist.

Die Aufgabe wird erfindungsgemäß nach den Merkmalen des Hauptanspruchs und des Nebenanspruchs gelöst.

Zweckmäßige Ausgestaltungen und Weiterbildungen der Erfindung sind in den Unteransprüchen angegeben.

Die mit der Erfindung erzielten Vorteile bestehen insbesondere darin, daß das Gehäuse des Bestrahlungsgerätes mit Überdruck betrieben wird, wobei die Strahlungsquelle allseitig von einem Luftpolster umschlossen ist, so daß sie auf die optimale Betriebstemperatur gehalten wird. Dabei wird die überschüssige Luft so auf das Filter geleitet, daß es die absorbierte Wärmestrahlung mit der Luft durch die vorhandenen Luftaustrittsöffnungen abführen kann. Aufgrund des vorgesehenen Sicherheitsabschaltsystems wird das Bestrahlungsgerät abgeschaltet, wenn das Filter zerstört wird und/oder der Lüfter ausfällt.

Bei einer Variante ist besonders vorteilhaft, daß eine Strahlungsquelle verwendbar ist, die im wesentlichen keine Ultraviolettstrahlung emittiert.

Weitere Vorteile und Einzelheiten der Erfindung sind nachstehend anhand von zwei in der Zeichnung dargestellten Ausführungsbeispielen und einer Alternative näher erläutert. Es zeigen:

Fig. 1 eine schematische Ansicht des Bestrahlungsgerätes mit Lüfter im Schnitt nach der Erfindung;

Fig. 2 einen Schaltplan für das Vorschaltgerät des Bestrahlungsgerätes nach der Erfindung;

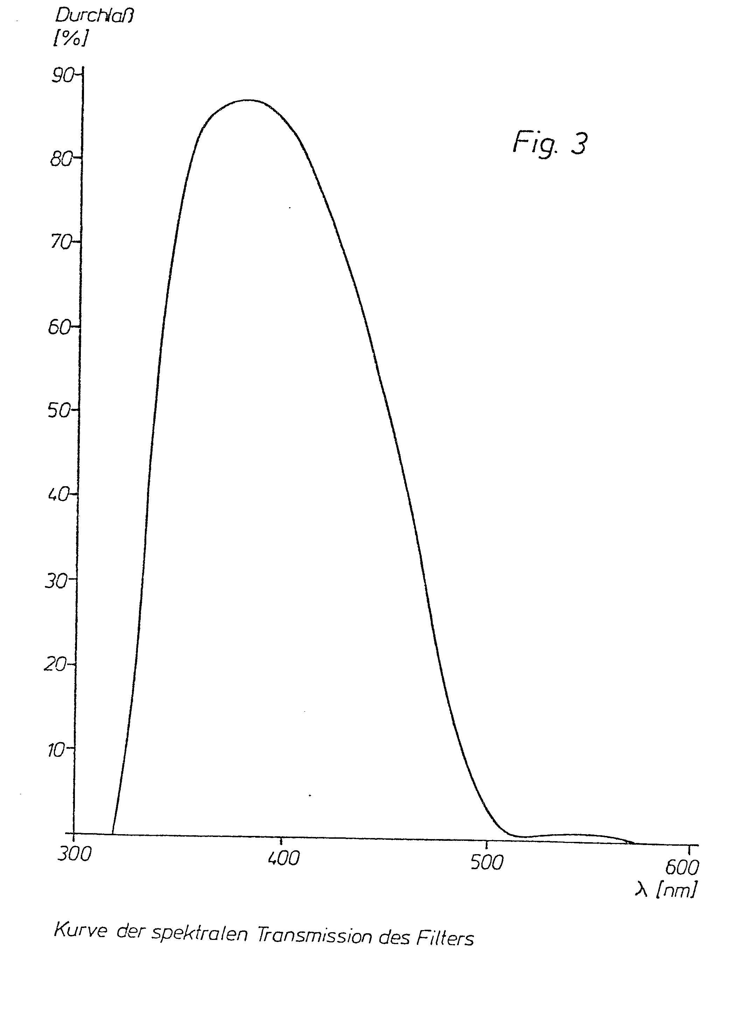

Fig. 3 eine Kurve der spektralen Transmission des Filters des Bestrahlungsgerätes nach der Erfindung und

Fig. 4 eine schematische Ansicht eines alternativen Ausführungsbeispiels des Bestrahlungsgerätes nach der Erfindung.

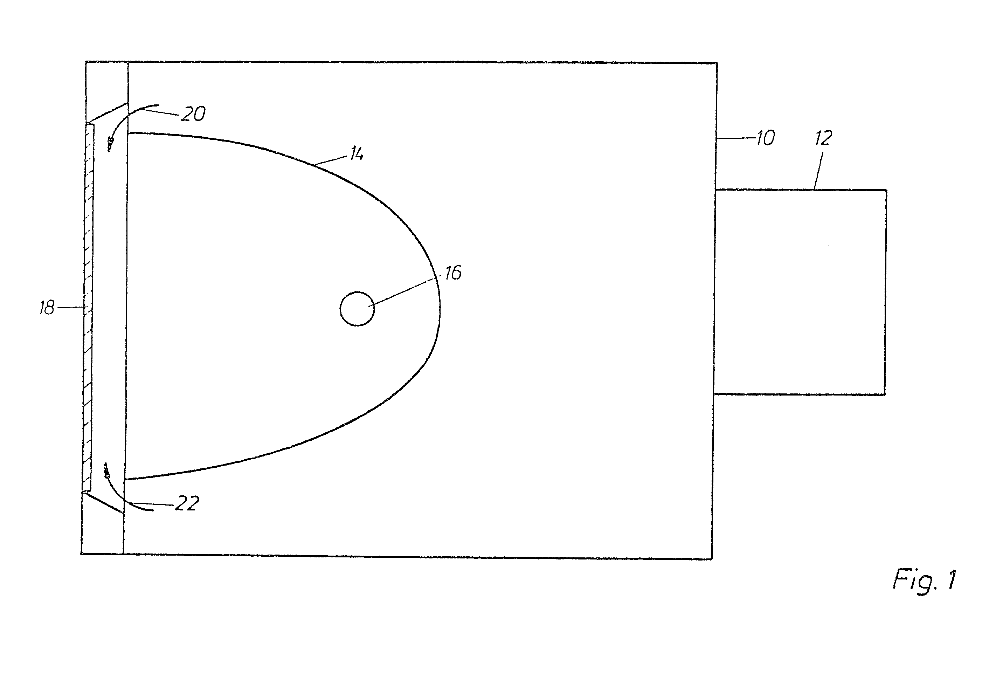

In Fig. 1 ist in schematischer Ansicht ein Gehäuse 10 mit einem Lüfter 12, vorzugsweise ein Radiallüfter, eines Bestrahlungsgerätes im Schnitt dargestellt. Im Inneren des Gehäuses ist deutlich ein Reflektor 14 zu erkennen, innerhalb dessen Bereichs eine Strahlungsquelle 16 angeordnet ist. Gegenüber dem Lüfter auf der linken Seite ist ein Frontfilter 18 zu erkennen, das vor dem Reflektor 14 angebracht ist.

Das Gehäuse 10 wird mit dem Lüfter 12 auf Überdruck gehalten. Durch entsprechend geführte Luftkanäle und Luftleitbleche wird die Strahlungsquelle 16 allseitig von einem Luftpolster umschlossen, so daß sie auf die optimale Betriebstemperatur von 850 bis 870°C gehalten wird. Dabei wird die überschüssige Luft über Lufteintrittsöffnungen 20, 22 so auf das Frontfilter 18 geleitet, daß die absorbierte Wärmestrahlung mit der Luft durch die vorhandenen Luftaustrittsöffnungen, die senkrecht zu den gezeigten Lufteintrittsöffnungen 20, 22
verlaufen, abgeführt wird. Die besonders empfindlichen
Quetschfüsse der Strahlungsquelle ragen aus dem Reflektorgehäuse heraus, so daß sie direkt von kühler Luft umschlossen sind und die höchst zulässige Temperatur von
150°C nicht überschreiten.

Der Reflektor 14 besteht vorzugsweise aus hochglanzeloxiertem Aluminium und ist in seiner Form so ausgebildet,
daß etwa die Hälfte der abgestrahlten Energie die Gesichtsfläche einer zu behandelnden Person bestrahlt, während die
andere Hälfte der abgestrahlten Energie auf den Oberkörper
der zu behandelnden Person übertragen wird. Damit ist gewährleistet, daß nach einer Bestrahlungsbehandlung kein
starker Kontrast zwischen der Gesichtsfläche und dem Oberkörper der zu behandelnden Person auftritt.

Mit dem Frontfilter 18 wird eine hohe Transmission dadurch
erreicht, daß ein Absorptionsfilter verwendet wird, das
gleichzeitig als UV-Kantenfilter ausgebildet ist. Da ein
solches Filter normalerweise bei etwa 650 Nanometer wieder
öffnet, sind durch ein Hochvakuumverfahren viele Reflektionsschichten aufgebracht worden, die den nahen Infrarotbereich bis etwa 1500 Nanometer weitgehend unterdrücken;
d. h. bis auf max. 1 % Transmission. Der Durchlaßbereich
dieses Filters liegt dann zwischen 320 und 480 Nanometer.
Dabei liegt das Maximum mit ca. 85 % zwischen 350 und 450
Nanometer, was aus Fig. 5 deutlich zu erkennen ist. Mit
Hilfe des besonderen Filters wird eine höhere bräunungswirksame Abstrahlung erzielt.

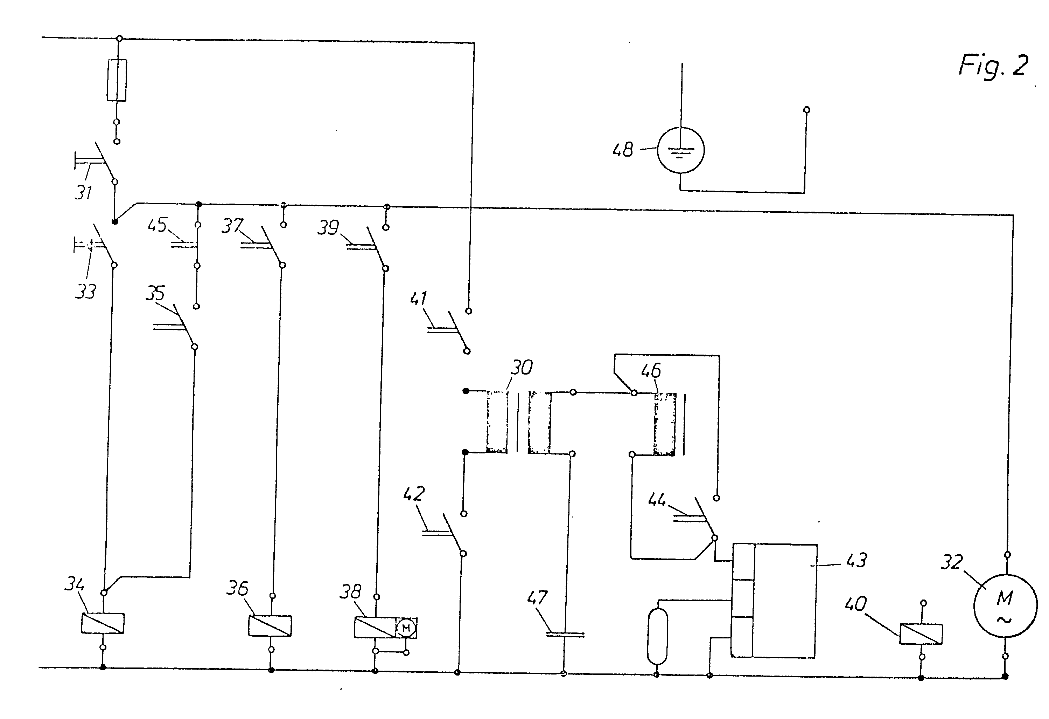

Das Bestrahlungsgerät weist auch ein Vorschaltgerät auf,
das wegen der Gewichtsersparnis vom Bestrahlungsgerät
getrennt angeordnet ist. Bei dieser Anordnung findet außerdem keine Aufheizung des Vorschaltgerätes durch das Bestrahlungsgerät statt. In Fig. 2 ist ein Schaltplan einer Schaltung für das Vorschaltgerät dargestellt, bei dem vorzugsweise ein Streufeldtransformator 30 verwendet wird. Die Verwendung des Streufeldtransformators hat den Vorteil, daß die Spannung für die Strahlungsquelle erhöht und damit der Strom für die Strahlungsquelle erniedrigt werden kann. Auf diese Weise kann die Lebensdauer der Strahlungsquelle erhöht werden. Gemäß Fig. 2 wird über einen Tastschalter 31 ein Motor 32 eines Lüfters für den Streufeldtransformator 30 an Spannung gelegt. Über einen Tastschalter 33, der in Serie zu dem Tastschalter 31 liegt, wird ein Relais 34 an Spannung gelegt, das anzieht und sich über einen Selbsthaltekontakt 35 hält. Als Folge des Anziehens von Relais 34 wird auch ein Relais 36 und ein Zeitrelais 38 jeweils über Kontakte 37 und 39 des Relais 34 an Spannung gelegt, wobei ein Ende dieser Kontakte zwischen den Tastschaltern 31 und 33 verbunden ist, während das andere Ende jeweils an das Relais 36 bzw. 38 geführt ist. Mit dem Einschalten des Motors 32 wird ebenfalls ein Relais 40 an Spannung gelegt, das über seine Kontakte 41, 42 seinerseits den Streufeldtransformator an Spannung legt. Über den Streufeldtransformator 30 und über einen Kontakt 44 des Relais 36 erhält die Strahlungsquelle über eine Startereinrichtung 43 die notwendige Spannung, um voll in Betrieb zu gehen. Wenn die im Zeitrelais 38 eingestellte Zeit abgelaufen ist und das Zeitrelais abfällt, wird ein Kontakt 45 des Zeitrelais im Selbsthaltestromkreis von Relais 34 geöffnet, so daß das Relais 34 und als Folge davon auch das Relais 36 abfallen. Damit öffnet auch der Kontakt 44 den Stromkreis für die Strahlungsquelle, die nunmehr über eine Schalteinrichtung 46, vorzugsweise ein Drossel, mit dem Streufeldtransformator 30 so verbunden ist, daß die Strahlungsquelle nunmehr im Teillastbetrieb gefahren wird. Am Streufeldtransformator ist noch ein Kompensationskondensator 47 angeschaltet. Darüber hinaus ist am Gehäuse noch eine Erdklemme 48 vorgesehen.

Mit der Teillasteinrichtung verfügt das Bestrahlungsgerät über eine Schaltung, die die Strahlungsquelle schonend auf kleine Betriebstemperatur fährt und somit die Lebensdauer der Strahlungsquelle erheblich erhöht. Dabei ist die Schaltung für die Teillasteinrichtung insbesondere als Sparlastschaltung ausgeführt, wodurch unnötige Aufheizzeiten zwischen den Bestrahlungsintervallen vermieden werden. Durch das vorgesehene Zeitrelais 38 kann die Bestrahlungszeit voreingestellt werden, so daß das Bestrahlungsgerät nach Ablauf der Bestrahlungszeit selbsttätig auf Teillast zurückgeschaltet wird. Infolge der getrennten Anordnung des Vorschaltgerätes vom Bestrahlungsgerät ist der Streufeldtransformator 30 nicht der hohen Umgebungstemperatur des Bestrahlungsgerätes ausgesetzt. Darüber hinaus kann der Streufeldtransformator gesondert gekühlt werden, so daß sich eine Einschaltdauer von 100 % ergibt.

Bei Ausfall des Lüfters 12 für das Bestrahlungsgerät würde die Strahlungsquelle überhitzt und somit auch das Frontfilter 18 zerstört werden. Daneben würde die zu behandelnde Person einer austretenden schädlichen Strahlung ausgesetzt, so daß dieser Vorfall unbedingt verhindert werden muß. Für diesen Zweck wurde eine Sicherheitszwangsabschaltung geschaffen, die das Bestrahlungsgerät sofort abschaltet, wenn das Frontfilter zerstört wird und/oder der Lüfter ausfällt. Für die Sicherheitszwangsabschaltung ist ein Windfahnenschalter so vorgesehen, daß die Zwangsabschaltung sowohl dann erfolgt, wenn der Lüfter ausfällt, als auch dann, wenn das Frontfilter zerstört wird. Zu diesem Zweck ist der Windfahnenschalter in dem Abluftkanal angeordnet. Infolge dieser bevorzugten Anordnung fällt der Windfahnenschalter einerseits dann sicher ab, wenn der Lüfter nicht mehr in vollem Betrieb ist, und andererseits, wenn das Frontfilter zerstört ist, so daß die Abluft dann direkt durch die Filteröffnung ins Freie gelangt und somit der Windfahnenschalter ebenfalls ausgelöst wird. In beiden Fällen wird das Bestrahlungsgerät sofort abgeschaltet.

Darüber hinaus ist eine Sicherheitsabschaltung gegen seitliches Kippen bzw. Umlegen des Bestrahlungsgerätes, ob beabsichtigt oder unbeabsichtigt, vorgesehen. Wenn nämlich die Strahlungsquelle vertikal betrieben wird, d. h. daß das Bestrahlungsgerät auf die Seite gelegt wird, tritt eine sofortige Zerstörung der Strahlungsquelle durch Gleichrichtung ein. Daher ist es angezeigt, eine Abschalteinrichtung zu schaffen, die das Bestrahlungsgerät sofort abschaltet, wenn das Bestrahlungsgerät um mehr als $10^o$ seitlich gekippt wird. Diese Abschalteinrichtung ist dadurch zu bewerkstelligen, daß ein Rüttelkontakt bzw. entsprechend angeordnete Quecksilberschalter angebracht sind.

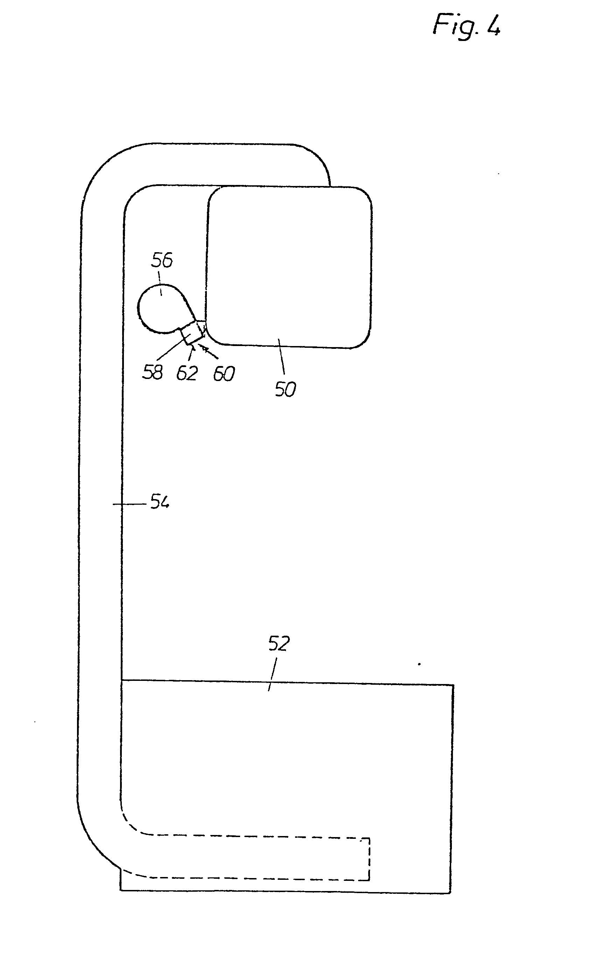

Gemäß Fig. 4 ist ein zweites Ausführungsbeispiel eines Strahlungsgerätes dargestellt, bei dem ein Strahlungsgehäuse 50 am oberen Schenkel eines quer liegenden U-förmigen Befestigungs- und Luftdruckkanals 54 angebracht ist, während auf dem unteren Schenkel des U-förmigen Rohres ein Schalt- und Liegeschrank 52 befestigt ist. Bei diesem Ausführungsbeispiel wird die Kühlluft für die Strahlungsquelle wie beim ersten Ausführungsbeispiel über die Strahlungsquelle und den Filter gedrückt. Alternativ kann die Kühlluft auch über die Strahlungsquelle und den Filter angesaugt werden. Bei diesem Ausführungsbeispiel befinden sich die Vorschaltgeräte im Luftstrom. Weiter wird die Luft vorzugsweise über ein Kühlsystem geführt, so daß auch hoch absorbierende Filter verwendet werden können, weil die Kühlung dieser Filter mit dieser Anordnung gewährleistet ist. Darüber hinaus wird durch entsprechend geführte Luftkanäle dieselbe Luft auch zur Kühlung bzw. zum Anblasen der zu bestrahlenden Person verwendet, so daß nicht unbedingt zusätzliche Lüfter dafür erforderlich sind. Vorteilhafterweise sind jedoch längsseitig am Strahlungsgehäuse 50 zwei Radialgebläse 56 angeordnet, die über einen Luftkanal 58 miteinander verbunden sind, der in Höhe des Filters und längsseitig am Strahlungsgehäuse verläuft. Der Luftkanal weist einen längs verlaufenden Luftschlitz 60 auf, an dem Luftleitbleche 62 so angebracht sind, daß die austretende Luft gleichmäßig auf die zu bestrahlende Person gerichtet ist. Des weiteren befinden sich auch die Schalteinheiten, wie z.B. Schalter und Kontrolleuchten, und die stromführenden Leitungen in dem Luftdruckkanal.

Vorzugsweise ist die Bestrahlungseinrichtung in mehreren Stufen schaltbar. Dabei ist die Bestrahlungseinrichtung grundsätzlich bis ca. 20 kW mit nur einer Strahlungsquelle und einem Reflektor ausgerüstet. Der Vorteil gegenüber den Bestrahlungsgeräten, die mit vier oder fünf Strahlungsquellen betrieben werden, liegt eindeutig darin, daß ein völlig gleichmäßig ausgeleuchtetes Bestrahlungsfeld erreicht wird. Darüber hinaus sind die Betriebskosten der Bestrahlungseinrichtung bei Anwendung von nur einer Strahlungsquelle wesentlich geringer, wobei das Auswechseln von nur einer Strahlungsquelle eine wesentliche Arbeitserleichterung bringt.

Bei dem zweiten Ausführungsbeispiel dient der Luftdruckkanal 54 gleichzeitig und vorzugsweise für die Anbringung des Strahlungsgehäuses 50.

Bei einem alternativen Ausführungsbeispiel ist eine Strahlungsquelle verwendbar, die im wesentlichen keine Ultraviolettstrahlung emittiert. Das wird mit einem Metallhalogenbrenner erreicht, der so dotiert ist, daß vorwiegend eine Strahlung im Bereich 380 bis 500 Nanometer emittiert wird. Dabei wird ein Filter angewendet, daß vorzugsweise die kürzerwellige Strahlung ab 380 Nanometer unterdrückt. Darüber hinaus sind bei diesem Ausführungsbeispiel im wesentlichen die gleichen Komponenten angeordnet wie bei den beiden vorher erwähnten Ausführungsbeispielen. Insbesondere ist also auch eine Kühlung der Strahlenquelle

und des Filters unter Überdruck vorgesehen, der von einem Lüfter erzeugt wird, der vorzugsweise als Radialgebläse ausgelegt ist.

Ein wesentlicher Vorteil bei dem alternativen Ausführungsbeispiel besteht darin, daß zum Bräunen der Haut auf einen Anteil an Ultraviolettstrahlung völlig verzichtet werden kann. Damit wird eine schädliche Wirkung auf die Haut im wesentlichen ausgeschaltet.

Ein besonderer Vorteil der bevorzugten Bestrahlungsgeräte ist darin zu sehen, daß die Pigmentierungsschwelle für die Sofortbräunung deutlich abgesenkt wird und bei unter fünfzig Sekunden liegt. Bei einer leistungsstarken Strahlungsquelle, beispielsweise 5 kW bei entsprechend kleinem Bestrahlungsfeld von etwa 50 x 60 cm, setzt eine Pigmentierung der Haut bereits nach zehn Sekunden ein.

PATENTANSPRÜCHE:

=====================================================================

1. Bestrahlungsgerät für medizinische und kosmetische Zwecke, das mindestens eine Ultraviolett-Strahlungsquelle und Vorschaltgeräte zum Betrieb der Strahlungsquelle und eine Filteranordnung enthält, die die kürzerwellige Strahlung ab 320 Nanometer unterdrückt, dadurch g e - k e n n z e i c h n e t , daß eine Kühlung der Strahlungsquelle (16) und des Filters (18) unter Überdruck betreibbar ist, daß die Strahlungsquelle im Einschaltmoment weniger als bei Vollast kühlbar ist und daß eine Sicherheitszwangsabschaltung gegen Kühlungsausfall und Filterbruch angeordnet ist.

2. Bestrahlungsgerät nach Anspruch 1, dadurch g e k e n n - z e i c h n e t , daß die Strahlungsquelle (16) als mit verschiedenen Metallen dotierter Metallhalogenbrenner ausgelegt ist.

3. Bestrahlungsgerät nach Anspruch 1 oder 2, dadurch g e - k e n n z e i c h n e t , daß die Strahlungsquelle (16) mit den Quetschfüßen in den von einem Lüfter (12) erzeugten Kühlluftstrom und außerhalb des Strahlenganges eines asymmetrisch ausgebildeten Reflektors (14) angebracht ist.

4. Bestrahlungsgerät nach Anspruch 1, dadurch g e - k e n n z e i c h n e t , daß die Sicherheitszwangsabschaltung gegen Ausfall der Kühlung und Bruch des Filters (18) mit einem im Abluftkanal angeordneten Windfahnenschalter aufgebaut ist.

5. Bestrahlungsgerät nach Anspruch 1, dadurch g e k e n n - z e i c h n e t , daß die Sicherheitszwangsabschaltung mit einem Rüttelkontakt oder Quecksilberschaltern gegen seitliches Kippen aufgebaut ist.

6. Bestrahlungsgerät nach Anspruch 1, dadurch g e k e n n - z e i c h n e t , daß das Filter (18) unter Überdruck so kühlbar ist, daß die Kühlluft durch eine Zwangsführung an den Seiten des Bestrahlungsgerätes von der Mitte des Filters herausdrückbar ist.

7. Bestrahlungsgerät nach Anspruch 6, dadurch g e - k e n n z e i c h n e t , daß die Kühlluft über die Zwangsführung von der zu bestrahlenden Person so wegführbar ist, daß die zu bestrahlende Person nicht mit dieser Luft in Berührung kommt.

8. Bestrahlungsgerät nach Anspruch 3, dadurch g e - k e n n z e i c h n e t , daß die Kühlluft über die Strahlungsquelle (16) ansaugbar ist.

9. Bestrahlungsgerät nach Anspruch 3, dadurch g e - k e n n z e i c h n e t , daß die Vorschaltgeräte im Ansaugluftstrom angeordnet sind.

10. Bestrahlungsgerät nach Anspruch 3, dadurch g e - k e n n z e i c h n e t , daß die angesaugte Luft über ein Kühlsystem leitbar ist.

11. Bestrahlungsgerät nach Anspruch 3, dadurch g e - k e n n z e i c h n e t , daß die angesaugte Luft über einen Luftdruckkanal (54) zur Kühlung des Filters und der Strahlungsquelle und zum Anblasen der zu behandelnden Person verwendbar ist.

12. Bestrahlungsgerät nach einem oder mehreren der Ansprüche 1 bis 10, dadurch g e k e n n z e i c h n e t , daß die Schalteinheiten und die stromführenden Leitungen für das Bestrahlungsgerät im Luftdruckkanal (54) angebracht sind.

13. Bestrahlungsgerät nach einem oder mehreren der Ansprüche 1 bis 12, dadurch g e k e n n z e i c h n e t , daß die Strahlungsquelle (16) in mehreren Stufen schaltbar ist.

14. Bestrahlungsgerät nach einem oder mehreren der Ansprüche 1 bis 13, dadurch g e k e n n z e i c h n e t , daß die Strahlungsquelle (16) bis zu großen Leistungen nur mit einem Brenner und einem Reflektor (14) ausgerüstet ist.

15. Bestrahlungsgerät nach Anspruch 3, dadurch g e k e n n z e i c h n e t , daß die Strahlungsquelle in einem Strahlungsgehäuse (50) angeordnet ist, das über den Luftdruckkanal (54) mit einem unter Überdruck stehenden Schalt- und Liegeschrank (52) so verbunden ist, daß die Überdruckluft über den Luftdruckkanal in das Strahlungsgehäuse zur Kühlung der Strahlungsquelle und des Filters führbar ist.

16. Bestrahlungsgerät nach Anspruch 15, dadurch g e k e n n z e i c h n e t , daß die Strahlungsquelle in einem Strahlungsgehäuse (50) mit zwei längsseitig angebrachten Radialgebläsen (56) angeordnet ist, die über einen in Höhe des Filters und längsseitig am Strahlungsgehäuse verlaufenden Luftkanal (58) verbunden sind, der einen längsseitig verlaufenden Schlitz (60) aufweist, an dem Luftleitbleche (62) so angebracht sind, daß die austretende Luft gleichmäßig auf die zu bestrahlende Person gerichtet ist.

17. Bestrahlungsgerät für medizinische und kosmetische Zwecke, das eine Strahlungsquelle und Vorschaltgeräte zum Betrieb der Strahlungsquelle und eine Filteranordnung enthält, die die kürzerwellige Strahlung unterdrückt, dadurch g e k e n n z e i c h n e t , daß für die Strahlungsquelle ein Filter angeordnet ist, das die kürzerwellige Strahlung ab 380 Nanometer unterdrückt, daß die Strahlungsquelle und das Filter unter Überdruck kühlbar sind und daß eine Sicherheitszwangsabschaltung gegen Kühlungsausfall und Filterbruch angeordnet ist.

18. Bestrahlungsgerät nach Anspruch 17, dadurch g e - k e n n z e i c h n e t , daß die Strahlungsquelle als mit verschiedenen Metallen dotierter Metallhalogenbrenner ausgelegt ist, der im wesentlichen eine Strahlung im Bereich von 380 bis 500 Nanometer emittiert.

19. Bestrahlungsgerät nach Anspruch 17 oder 18, dadurch g e k e n n z e i c h n e t , daß die Strahlungsquelle im Einschaltmoment weniger als bei Vollast kühlbar ist.

20. Bestrahlungsgerät nach einem der Ansprüche 17 bis 19, dadurch g e k e n n z e i c h n e t , daß die Strahlungsquelle in einem Strahlungsgehäuse (50) angeordnet ist, das über den Luftdruckkanal (54) mit einem unter Überdruck stehenden Schalt- und Liegeschrank (52) so verbunden ist, daß die Überdruckluft über den Luftdruckkanal in das Strahlungsgehäuse zur Kühlung der Strahlungsquelle und des Filters führbar ist.

Fig.1

Fig. 2

0026239

Durchlaß
[%]

90

80

70

60

50

40

30

20

10

*Fig. 3*

300          400          500          600

λ [nm]

Kurve der spektralen Transmission des Filters

*Fig. 4*

56

58  62  60

50

54

52

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 714 724 (MUTZHAS)<br>* Seite 5, Zeile 1 - Seite 6, Zeile 11 * | 1,3,7, 8,10, 11,14, 19 |
| | -- | |
| | DE - C - 900 118 (HANAU)<br>* Seite 2, Zeilen 30-76; Figur 1 * | 1,6-8, 10,11 |
| | -- | |
| | DE - A - 2 152 933 (MUZ)<br>* Seite 5, Zeile 18 - Seite 6, Zeile 9 * | 1-3,14, 16 |
| | -- | |
| | DE - A - 2 008 490 (MUZ)<br>* Seite 3, Zeile 14 - Seite 4, Zeile 20; Figuren * | 1,10, 18 |
| | -- | |
| | US - A - 3 986 513 (STUHL)<br>* Spalte 2, Zeile 58 - Spalte 3, Zeile 10; Spalte 3, Zeilen 37-46 * | 1,16- 18 |
| | ---- | |

**KLASSIFIKATION DER ANMELDUNG (Int.Cl 3)**

A 61 N 5/06

**RECHERCHIERTE SACHGEBIETE (Int. Cl 3)**

A 61 N 5/06
        5/08

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsatze

E: kollidierende Anmeldung

D: in der Anmeldung angefuhrtes Dokument

L: aus andern Grunden angefuhrtes Dokument

&: Mitglied der gleichen Patent- familie, übereinstimmendes Dokument

Der vorliegende Recherchenbericht wurde fur alle Patentanspruche erstellt.

| Recherchenor. Den Haag | Abschlußdatum der Recherche 19-05-1980 | Prüfer BIJN |
|---|---|---|

EPA form 1503.1   06.78